# EUROPEAN PATENT SPECIFICATION

(11) **EP 2 318 092 B1**
(45) Date of publication and mention of the grant of the patent: **29.11.2017**
(21) Application number: 08808159.1
(22) Date of filing: 12.08.2008
(51) Int. Cl.: A61N 1/362, A61N 1/32

(54) **INTERFERENTIAL NON INVASIVE TEMPORARY PACER**
INTERFERENTIELLER NICHT-INVASIVER TEMPORÄRER SCHRITTMACHER
STIMULATEUR TEMPORAIRE NON INVASIF INTERFÉRENTIEL

(30) Priority: 22.07.2008 IN CH17512008
(43) Date of publication of application: 11.05.2011
(73) Proprietor: Lakshmanan, Narayanan, Chennai 600059 (IN)
(72) Inventor: Lakshmanan, Narayanan, Chennai 600059 (IN)
(74) Representative: Patentbüro Paul Rosenich AG
(86) International application number: PCT/IN2008/000508
(87) International publication number: WO 2010/010567

(56) References cited:
- WO-A2-2004/064915
- US-A- 3 895 639
- US-A- 4 576 170
- US-A- 5 324 317
- US-A- 5 330 506
- US-A- 5 431 688
- US-A- 5 776 173
- US-A1- 2002 068 961
- US-A1- 2007 299 895

## Description

This invention relates to the Medical and in particular to non invasive cardiac pacing. In particular, it relates to Temporary Emergency Pacing to treat sudden cardiac arrest. It also offers solution to existing cutaneous nerve stimulation as well as skeletal muscle stimulation which is associated with the discomfort experienced by the patient in the existing method. The Noninvasive Temporary Pacing (popularly known as NTP) is well known technique used in emergency to manage acute bradycardia. Electrical current is passed from an external pulse generator via a conducting cable and externally applied, self adhesive electrodes through chest wall and heart. It is a valuable tool as initial mode of cardiac pacing for brady systolic arrest situations and prophylactic pacing applications. This technique is advantage that any physician can use it with very little training and it is non invasive method and easy of application during emergency.

For emergency pacing modalities, transcutaneous cardiac pacing is a valuable technique as it can be quickly safely and easily initiated by minimally trained personnel. It may be effective when endocardial pacing fails or is contraindicated.

Zoll et. al had proved that stimulating the ventricle would cause it to contract and that such stimulation could be effective through intact chest wall.

US 5 431 688 shows an apparatus for transcutaneous electrical cardiac pacing with two pair of electrodes aranged so that current paths between electrodes in each pair intersect at the patient's heart. US 5 330 506 shows a cardiac pacing apparatus with a plurality of electrode pairs, wherein the pacing currents delivered to the electrode pairs add to produce a total cardiac pacing current of sufficient magnitude to cause contraction of a heart muscle through which the pacing currents flow.

Several interferential stimulator devices have previously been described. US2007/0299895, U.S. Pat No 5,776,173, US Pat No 5,324,317 and US2002/068961 show interferential stimulators for stimulating individual nerves or muscles of the body. Transcutaneous cardiac pacing produces depolarization of myocardial tissue by pulsed electrical current conducted through the chest between electrodes adherent to the skin. The self-adhesive surface patch electrodes are large in area - typically 8cm in diameter. Currently available transcutaneous pacing generators may come as stand-alone units or be incorporated in to external defibrillators. Transcutaneous pacing generators should feature asynchronous and demand pacing modes, a built in ECG display that filters the large pacing artifact, widely adjustable settings for pacing rate and current output. Available units provide up to 200mA of current and use a rectangular or truncated exponential pulse waveform of 20 to 40 msec duration. The long pulse width permit the lowest pacing thresholds while minimizing the stimulation of skeletal muscle and cutaneous nerves. To initiate transcutaneous pacing, the patch electrodes are secured anteriorly and posteriorly to the chest wall.

In emergency bradyasystolic situations or with unconscious patients, transcutaneous pacing should be initiated in the asynchronous mode and at maximal current output to ensure ventricular capture. Chest compressions may be performed directly over the electrodes without disruption of pacing or conduction of significant electrical current to medical personnel. Cardiac capture is suggested by the appearance of depolarization artifacts following the pacing stimuli, but capture must be confirmed by palpation of a pulse or using oximeter. Once capture is documented, the current may be decreased gradually until loss of capture defines the pacing current threshold.

Also, it is well known that this technique produces painful muscular stimulation, variable incidence of cardiac capture and poor patient tolerance. This discomfort often intolerable restricts external stimulation to use in unconscious patients or to brief use in desperate situations. Because of the pain and supposed ineffectiveness of noninvasive pacing, endocardial pacing was generally preferred even for temporary use, in both emergency and standby situations.

The undesirable side effects of this present technique can be summarized as follows:
1. The current output that produces pain which is highly variable among individuals. Majority of patients cannot be paced a tolerable level of discomfort.
2. The pain results from simulation of cutaneous afferent nerves and intense pacing induced skeletal muscle contraction.

Also, there have been many proposals to overcome this disadvantage like using ramp waveform etc., instead of pulsed current output but these proposals only reduced or likely to reduce the undesirable effects. It cannot overcome the disadvantage of unnecessary pacing induced stimulation of skeletal muscle and cutaneous afferent nerves.

It is therefore an object of invention is to provide an apparatus for stimulating myocardium using interferential methodology. Present invention presents a unique approach to the above disadvantage by which it overcomes unnecessary pacing induced stimulation of skeletal muscle and cutaneous afferent nerves. This invention will popularize in emergency settings due to easy of application and minimal training. Also this invention opens up more usage with proper trials only to stimulate a particular area of myocardium.

### BRIEF SUMMARY OF THE INVENTION

A technique of non invasive cardiac stimulation is disclosed here such that it is very useful in sustaining heart beat and cardiac output in cases of bradycardia, low blood pressure and cardiac arrest. It also overcomes the disadvantage mentioned with non invasive temporary pacer of the current art. The present invention provides an apparatus for stimulating cardiac muscles by external non invasive technique as defined by claim 1.

Preferred embodiments are defined by the dependent claims.

Aspects, methods, and techniques which do not fall within the scope of the appended claims do not form part of the present invention.

The heart of the invention is that it uses the known phenomena of high frequency currents do not produce neuro-muscular stimulation. It is a known fact that, frequency above 1 KHz do not produce any stimulation. When two such high frequency currents of slightly different frequency, passed intersecting at the area of interest as shown in fig (3) then in that focal point (area of intersection) due to interference it produces a pulsating low frequency pulses which will stimulate the focal point. This technique is the basic of heart of invention. This technique produces no stimulation at the current entry point due to high frequency and hence there will be no transcutaneous stimulations at the site of electrodes. Whereas by planning the two circuits of high frequency currents of such a way the intersection of currents exactly located in the ventricular region of myocardium then due to integration of anti-phasic currents produces a perfect stimulus to myocardium in isolation. This technique of stimulating myocardium in isolation without other neuro-muscular system getting stimulated, presents a novel and powerful technique to pace the heart non invasively.

Interferential electrical stimulation's is a unique way of effectively delivering therapeutic stimulus to cardiac tissues. Conventional pacemakers use discrete electrical pulses. However, Interferential pacemaker use a fixed carrier frequency of 4,000 Hz per second in the first channel and a second channel of with adjustable frequency of 4001-4002 Hz per second. When the two channel currents combine (heterodyne), they produce the desired signal pulsed beat (Interference beat). Interferential stimulation is concentrated at the point of intersection of currents (between the electrodes). This concentration occurs deep in the tissues as well as at the surface of the skin. Conventional pacemakers deliver most of the stimulation directly under the electrodes. Thus, with Interferential pacemaker, current perfuses to greater depths and over a larger volume of tissue than other forms of electrical therapy. When current is applied to the skin, capacitive skin resistance decreases as pulse frequency increases. For example, at a frequency of 4,000 Hz (Interferential unit) capacitive skin resistance is eighty (80) times lower than with a frequency of 50 Hz. Thus, Interferential current crosses the skin with greater ease and with less stimulation of cutaneous receptors allowing greater patient comfort during electrical stimulation. In addition, because medium-frequency (Interferential) current is tolerated better by the skin, the dosage can be increased, thus improving the ability of the Interferential current to permeate tissues and allowing easier access to deep cardiac structures. This explains why Interferential current is most suitable for treating patients non invasively.

More specifically, the present invention uses two sets of electrodes positioned such that the target area myocardium is located straight intersection of these four electrodes. Diagonally opposite pair of electrodes is connected to one circuit. An isolated high frequency current is passed through the electrode in anti phasic manner.

In accordance with one aspect of invention the base carrier frequency of the current can be anything above 10 kHz and the channel phase relationship can be varied slightly with reference to one channel to other so that interferential stimulus, stimulates the myocardium. In accordance with still another aspect of invention, by suitably placing the electrodes, the target stimulus area can be selected such as atrium or ventricle of the heart. This offers the physician better control of pacing and sustain life with better cardiac output.

In accordance with yet another aspect of invention the same pacing electrodes can be used to pick up ECG which will help in avoiding placing separate ECG electrodes for the ECG monitoring. This saves lot of time in cardiac medical emergency.

It will be appreciated that the disclosed apparatus is advantageous in that it allows only myocardium getting stimulated and hence there is no pain or muscular stimulation and hence well tolerated by the patient. To assist with understanding the invention, reference will now be made to the accompanying drawings, which show one example of invention.

The foregoing aspects and many of the attendant advantages of this invention will become more readily appreciated as the same becomes better understood by reference to the detailed description, when taken in conjunction with the accompanying drawings.
Fig 1 is a prior art of using Non invasive Temporary Pacer showing the pad placement. The figure 1 a shows Anterior / Lateral electrode placement and 1b shows Anterior / Posterior electrode placement.
Fig 2. illustrates the principle of Interferential Current.

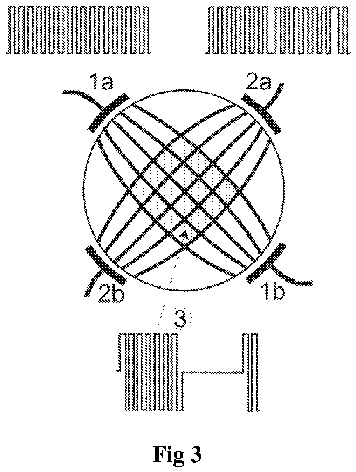

Fig 3. illustrates the tissue stimulation using Interferential Current.

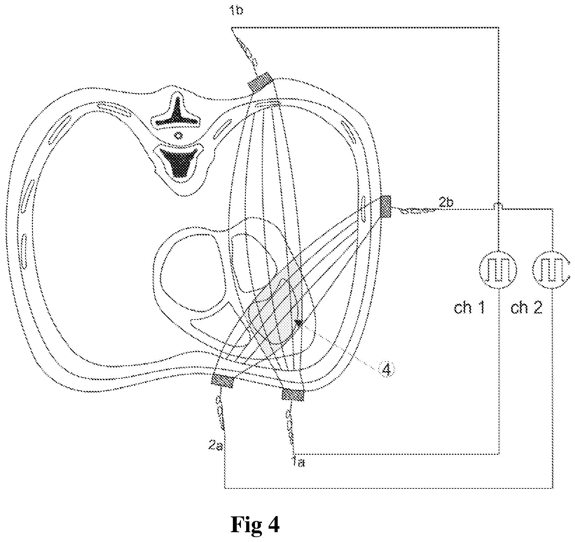

Fig 4. illustrates the inventive step of passing high frequency current and how the target area getting stimulated.

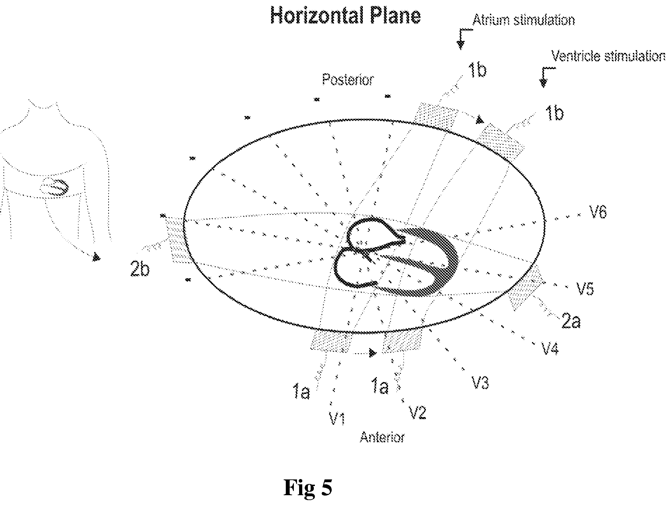

Fig 5 illustrates the electrode placement position to stimulate different areas of myocardium
Fig 6 illustrates method of electrode placement for Interferential Pacing

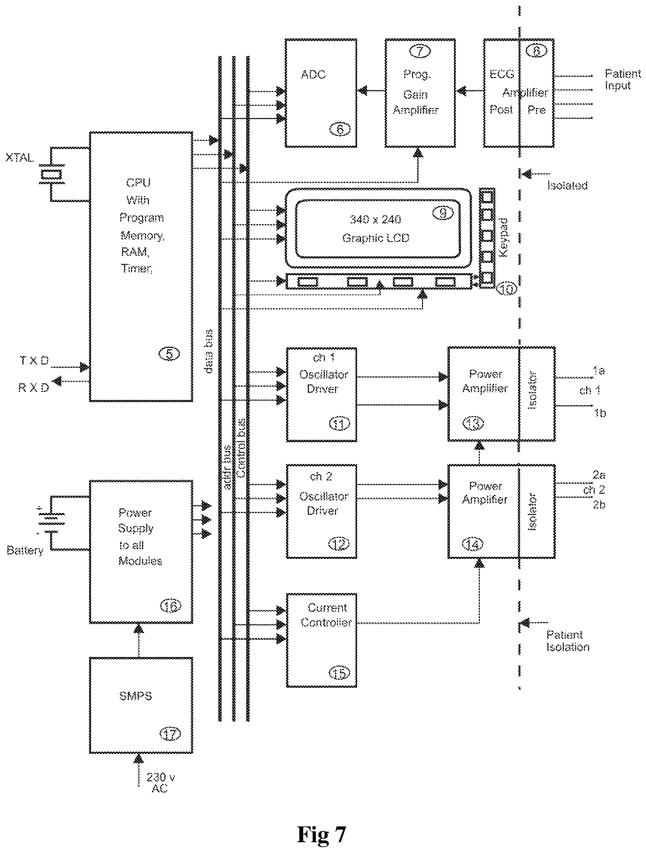

Fig 7 illustrates the block diagram of the interferential non invasive pacing.

The Figs (2) to Fig (6) clearly explains the technique used to stimulate the myocardium the heart using two high frequency currents. This technique of stimulation overcomes all serious disadvantages of prior art. Also user can adjust the stimulation area to get the desired effect.

The general operation of the preferred embodiment of the present invention is illustrated in Fig (4). This differs from the prior art of NTP that it requires four conducting electrodes along with high frequency currents as shown. The set of electrodes 1a and 1b constitute one circuit and similarly 2a and 2b constitute another isolated circuit. When high frequency currents are passed, the currents heterodyne at the intersection point and resultant beat impulse is produced in the target area (4). Fig (4) explicitly explains how cardiac stimulation can be achieved by using interferential technique. The location of the pads and its usefulness to select an area of stimulation is described in Fig (5). By moving set of opposite electrodes 1a & 1b, one can achieve any target area stimulation. May be Atrium or Ventricle can be targeted. This feature will be helpful to improve the cardiac output by an experienced physician by properly stimulating the atrium or ventricle. Placement of electrodes is the key to the selection of area in the myocardium.

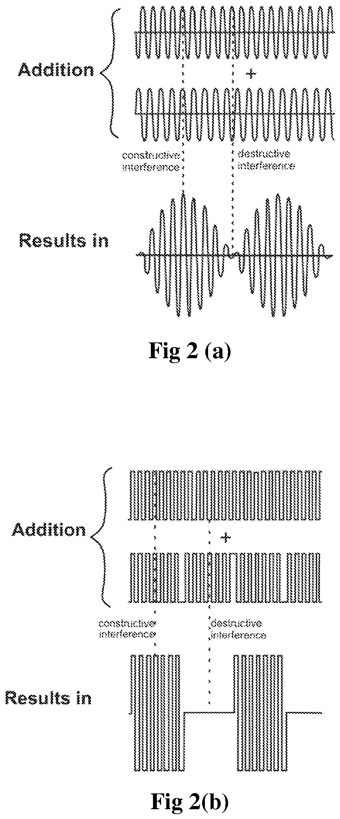

Fig (2a) and 2(b) explains the basic working of Interferential currents. Fig 2(a) illustrates how two channels of Isolated High frequency currents when crosses it heterodyne and produces beating frequency. In Fig (2b) illustrates how out of phase pulsed high frequency produces the gated pulses. This explains the basic principle behind the myocardium stimulation by using High frequency.

Fig (3) illustrates how the actual interferential current from two channels from properly positioned electrodes produces stimulation in the intersection path area (3). Fig (1) illustrates how in prior art the electrodes are positioned.

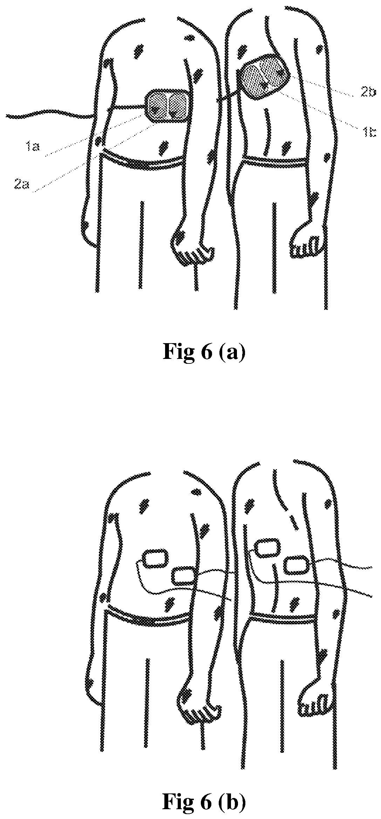

Fig (6) illustrates the electrode placement for interferential pacing. Depending on the area of myocardial stimulation the electrode placements will vary from one end to another in the thorax. There is numerable ways of electrode placement possible to achieve different effects. Meanwhile the fig 6(a) illustrates using integrated 2 electrodes like prior art but the one piece of electrode will contain two circuits as shown and with two electrodes two circuits are established to produce interferential currents. This may be easily achievable by using 4 separate electrodes as shown in Fig 6(b). With Fig 6 (b) scheme one can adjust and attain stimulation precisely at different parts of myocardium.

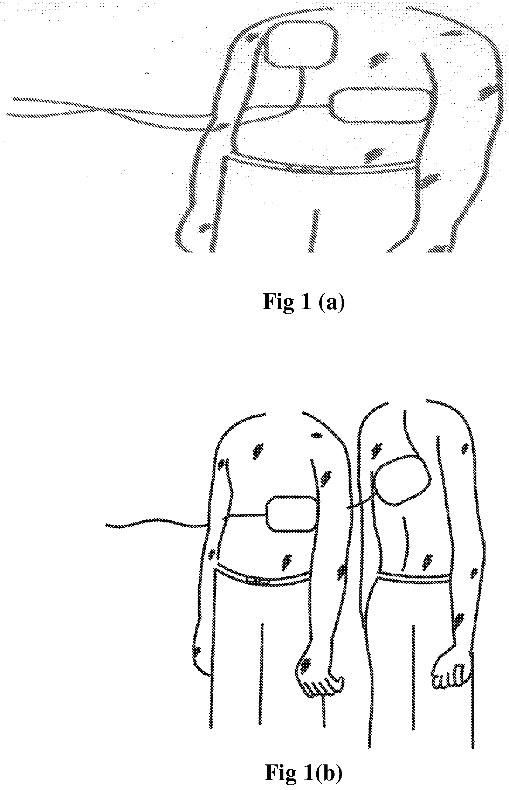

Fig (1) illustrates the prior art of Non Invasive Temporary pacing using two big electrodes. Two methods of electrode placement are popular as shown in Fig 1 (a) Anterior / Lateral placement and Fig 1 (b) Anterior / Posterior placement.

While the preferred embodiments of the invention has been illustrated and described, it will be apparent that various changes can be made therein without departing from the scope of the invention as defined by the appended claims. For example, the Fig (5) electrode positioning is shown in one axis whereas electrode movement can be made in another axis. Also the movement itself be made using an automatic tool. Consequently, within the scope of the appended claims, it will be appreciated that the invention can be practiced otherwise than as specifically described herein.

Fig (7) illustrates the Block Diagram of Interferential Noninvasive Pacing Device. The CPU (5) is the Master of the Device. It is functionally responsible for the acquisition and processing of ECG, Displaying of ECG, Generating time base for the two channels, controlling the modes of pacing and strength of pacing currents. A microcontroller with inbuilt Program memory, RAM, Timer and I/O Ports is used. The ADC (6) digitize the processed ECG signal fed by Programmable Gain Amplifier (7). The Programmable Gain amplifier is controlled by the CPU for the Gain. The Input ECG amplifier (8) consists of Pre and post amplifiers. The Pre amplifier is fully electrically isolated for the patient safety.

The ECG and Pacing Pulse is displayed as two traces. ECG trace is displayed above and the Pace stimulation pulse is displayed as second trace below. This allows the user to instantly compare the two traces and conclude the cardiac capture due to non invasive stimulation. The display is of Graphic LCD / TFT screen (9) sufficient enough to display two traces. The CPU (5) directly writes in to graphic display and maintains the display content. Also the Keyboard (10) is interfaced to CPU (5). User can program the stimulus frequency, its current strength etc., using this keyboard.

Also the CPU (2) is connected to heart of the Pacer consisting of two channels viz., Channel 1 (11) and Channel 2 (12). Each channel is programmed to base frequency of say 4000Hz and either one of them can be phase or frequency shifted by the CPU (5) as per the user program programmed earlier. The identical high frequency signals are getting amplified by the respective power amplifiers (13) and (14). The Power amplifier is coupled with Transformer isolator that the output current is fully isolated and independent. The current strength is varied by the CPU (5) directly by using a current controller (15) which biases the Power amplifier to deliver the programmed output current.

Depending on the setting of the Pace frequency the CPU (5) outputs high frequency pulsed currents via two channels. But the waveforms of the channel are modulated in such a way that the beat frequency process the stimulus pulse at the target area.

In this preferred embodiment it will be appreciated that the both channels are closely controlled by the CPU and the output current is isolated electrically by means of magnetic isolation.

Also it will be appreciated that the Pacer can be put into Asynchronous Mode or in Demand Mode. Again the outputting beat frequency is software controlled accordingly to the mode set by the user.

Power supply section (16) is a DC-DC Converter with battery charger. It supplies all the power requirements of blocks. It also charges the battery whenever the Mains power is connected and present. The Mains Power is converted into DC by SMPS (17).

While the preferred embodiments of the invention has been illustrated and described, it will be apparent that various changes can be made therein without departing from the scope of the invention as defined by the appended claims. For example, the generation of the bi-phasic high frequency currents can be devised by many means.

## Claims

1. An apparatus for stimulating cardiac muscles by external non invasive technique comprising:
a) a generator of isolated high frequency current of two channels of same frequency but variable phase;
b) an applied part with at least two pair of electrodes configured to be placed externally to thorax of the human body in such a manner that the output current emanating from the pair of electrodes crisscrosses the heart, myocardium or part of myocardium
c) wherein the said two pairs of electrodes, one pair for each channel position are adapted to be adjusted in a manner that target area, heart, myocardium or part of myocardium is located at straight intersection of these four electrodes and each diagonally opposite pair of electrodes is connected to one circuit;
d) wherein the said currents applied through electrodes are configured to produce an interferential pulsed pacing stimulus, in the crisscrossed area of the current, which stimulates the heart, myocardium, or part of myocardium;
e) wherein the said generator has two modes of operation, asynchronous and demand mode;
f) wherein the said pulsed stimulus repetition rate and intensity of current are selectable by the user;
g) wherein the said current generated by the generator is of a square or pulse modulated wave with a base frequency of at least 1 KHz;
h) wherein the interferential pulsed pacing stimulus has a pulse repetition frequency, configured to effect cardiac capture;
i) wherein the interferential pulsed pacing stimulus has an output current configured to effect cardiac capture;
j) wherein the interferential pulsed pacing stimulus is biphasic;
k) wherein the said generator is configured to sense the ECG (electrocardiogram) so that the cardiac capture is documented;
l) wherein the said generator is configured to be controlled by the sensing the patient's ECG (electrocardiogram) so that both asynchronous and demand pacing modes are achieved.

2. An apparatus as claimed in claim 1, **wherein** the electrodes of the applied part are adapted to be steered mechanically over the chest to attain the selective stimulation of particular area of heart, part of myocardium.

3. An apparatus claimed in claim 1, **wherein** the electrodes of opposite channel of the applied part are integrated into one electrode.

4. An apparatus claimed in claim 1, **wherein** the electrodes are adapted to be manually or automatically, moved to select a target area of stimulation.

5. An apparatus claimed in claim 1 wherein, the pacing electrodes are adapted for sensing the ECG from the pacing electrodes and the apparatus is configured to process and display the ECG (electrocardiogram) in an LCD Monitor (9) and without use of any further electrodes for monitoring the ECG.

## Patentansprüche

1. Vorrichtung zum Stimulieren der Herzmuskeln durch eine externe nicht-invasive Technik, welche Folgendes umfasst:
a) einen Generator von isoliertem Hochfrequenzstrom mit zwei Kanälen der gleichen Frequenz, aber variabler Phase;
b) ein aufgebrachtes Teil mit mindestens zwei Paaren von Elektroden, das ausgebildet ist, extern derart auf den Thorax des menschlichen Körpers aufgebracht zu werden, dass der von dem Paar von Elektroden ausgehende Ausgangsstrom das Herz, das Myokardium oder einen Teil des Myokardiums kreuz und quer durchläuft;
c) wobei die zwei Paare von Elektroden, je ein Paar für jede Kanalposition, ausgebildet sind, in einer Weise eingestellt zu werden, dass das Zielgebiet, Herz, Myokardium oder Teil des Myokardiums, sich am geraden Schnittpunkt dieser vier Elektroden befindet, und jedes diagonal gegenüberliegende Paar von Elektroden mit einem Stromkreis verbunden ist;
d) wobei die durch die Elektroden aufgebrachten Ströme ausgebildet sind, eine interferentiell gepulste Schrittmacherstimulation in dem gekreuzten Bereich des Stroms zu erzeugen, die das Herz, das Myokardium oder einen Teil des Myokardiums stimuliert;
e) wobei der Generator zwei Betriebsarten aufweist, einen asynchronen und einen Demand-Modus;
f) wobei die Wiederholungsrate der gepulsten Stimulation und die Stromstärke vom Benutzer wählbar sind;
g) wobei der von dem Generator erzeugte Strom eine Rechteck- oder pulsmodulierte Welle mit einer Grundfrequenz von mindestens 1 KHz darstellt;
h) wobei die interferentiell gepulste Schrittmacherstimulation eine Pulswiederholungsfrequenz aufweist, die ausgebildet ist, eine Erfassung des Herzsignals zu bewirken;
i) wobei die interferentiell gepulste Schrittmacherstimulation einen Ausgangsstrom aufweist, der ausgebildet ist, eine Erfassung des Herzsignals zu bewirken;
j) wobei die interferentiell gepulste Schrittmacherstimulation zweiphasig ist;
k) wobei der Generator ausgebildet ist, das EKG (Elektrokardiogramm) zu erfassen, so dass die Erfassung des Herzsignals dokumentiert wird;
l) wobei der Generator ausgebildet ist, durch das Erfassen des EKGs (Elektrokardiogramms) des Patienten gesteuert zu werden, so dass sowohl der asynchrone als auch der Demand-Stimulationsmodus erzielt wird.

2. Vorrichtung nach Anspruch 1, wobei die Elektroden des aufgebrachten Teils ausgebildet sind, mechanisch über die Brust bewegt zu werden, um die selektive Stimulation von bestimmten Bereichen des Herzens oder eines Teils des Myokardiums zu erreichen.

3. Vorrichtung nach Anspruch 1, wobei die Elektroden der gegenüberliegenden Kanäle des aufgebrachten Teils in eine Elektrode integriert sind.

4. Vorrichtung nach Anspruch 1, wobei die Elektroden ausgebildet sind, manuell oder automatisch bewegt zu werden, um einen Zielbereich der Stimulation auszuwählen.

5. Vorrichtung nach Anspruch 1, wobei die Stimulationselektroden ausgebildet sind, das EKG von den Stimulationselektroden zu erfassen, und die Vorrichtung ausgebildet ist, das EKG (Elektrokardiogramm) auf einem LCD-Monitor (9) und ohne Verwendung weiterer Elektroden zum Überwachen des EKGs zu bearbeiten und anzuzeigen.

## Revendications

1. Appareil pour stimuler les muscles cardiaques par une technique non invasive externe comprenant :
a) un générateur de courant à haute fréquence isolée de deux canaux de même fréquence mais de phase variable ;
b) une partie appliquée avec au moins deux paires d'électrodes configurées pour être placées de manière externe au thorax du corps humain de telle manière que le courant de sortie émanant de la paire d'électrodes couvre le coeur, le myocarde ou une partie du myocarde
c) où lesdites deux paires d'électrodes, une paire pour chaque position de canal sont adaptées pour être ajustées de manière telle que la zone cible, le coeur, le myocarde ou une partie du myocarde est localisée à l'intersection linéaire de ces quatre électrodes et chaque paire d'électrodes diagonalement opposée est connectée à un circuit ;
d) où lesdits courants appliqués par les électrodes sont configurés pour produire un stimulus de rythme impulsé interférentiel, dans la zone couverte du courant, qui stimule le coeur, le myocarde ou une partie du myocarde ;
e) où ledit générateur a deux modes d'opération, asynchrone et en mode de demande ;
f) où ledit taux de répétition de stimulus impulsé et l'intensité du courant sont sélectionnables par l'utilisateur ;
g) où ledit courant produit par le générateur est d'une onde modulée carrée ou à impulsion avec une fréquence de base d'au moins 1 KHz ;
h) où le stimulus de rythme impulsé interférentiel a une fréquence de répétition d'impulsion, configurée pour effectuer une capture cardiaque ;
i) où le stimulus de rythme impulsé interférentiel a un courant de sortie configuré pour effectuer une capture cardiaque ;
j) où le stimulus de rythme impulsé interférentiel est biphasique ;
k) où ledit générateur est configuré pour détecter l'ECG (électrocardiogramme) de sorte que la capture cardiaque est documentée ;
l) où ledit générateur est configuré pour être contrôlé par la détection de l'ECG du patient (électrocardiogramme) de sorte que les deux modes de rythme asynchrone et de demande sont obtenus.

2. Appareil selon la revendication 1, dans lequel les électrodes de la partie appliquée sont adaptées pour être mécaniquement guidées sur le buste pour atteindre la stimulation sélective d'une zone particulière du coeur, d'une partie du myocarde.

3. Appareil selon la revendication 1, dans lequel les électrodes de canal opposé de la partie appliquée sont intégrées dans une électrode.

4. Appareil selon la revendication 1, dans lequel les électrodes sont adaptées pour être manuellement ou automatiquement déplacées pour sélectionner une zone cible de stimulation.

5. Appareil selon la revendication 1 dans lequel, les électrodes de rythme sont adaptées pour détecter l'ECG des électrodes de rythme et l'appareil est configuré pour traiter et afficher l'ECG (électrocardiogramme) sur un moniteur LCD (9) et sans utilisation d'aucune autre électrode pour suivre l'ECG.
